## Europäisches Patentamt

## European Patent Office

## Office européen des brevets

(19)

(11) Veröffentlichungsnummer: **0 216 872**
**B1**

# (12) EUROPÄISCHE PATENTSCHRIFT

(45) Veröffentlichungstag der Patentschrift: **19.09.90**

(21) Anmeldenummer: **86902269.9**

(22) Anmeldetag: **09.04.86**

(86) Internationale Anmeldenummer:
**PCT/AT86/00029**

(87) Internationale Veröffentlichungsnummer:
**WO 86/06060 23.10.86 Gazette 86/23**

(51) Int. Cl.⁵: **C 02 F 3/08**, C 02 F 3/18,
B 01 D 19/00, C 12 M 1/02

(54) **EINRICHTUNG ZUM KONTINUIERLICHEN EINBRINGEN BZW. AUSBRINGEN VON GASEN IN BZW. AUS FLÜSSIGKEITEN.**

(30) Priorität: **09.04.85 AT 1067/85**

(43) Veröffentlichungstag der Anmeldung:
**08.04.87 Patentblatt 87/15**

(45) Bekanntmachung des Hinweises auf die
Patenterteilung:
**19.09.90 Patentblatt 90/38**

(84) Benannte Vertragsstaaten:
**BE CH DE FR GB IT LI LU NL SE**

(56) Entgegenhaltungen:
**DE-A-2 625 230**
**FR-A-1 320 157**
**FR-A-1 563 514**
**FR-A-2 264 584**
**US-A-4 211 647**

(73) Patentinhaber: **VOEST-ALPINE**
**Aktiengesellschaft**
**Muldenstrasse 5**
**A-4020 Linz (AT)**

(72) Erfinder: **CVITAS, Vilim**
**Voltastrasse 29**
**A-4040 Linz (AT)**
Erfinder: **FALTEJSEK, Karl**
**Lüfteneggerstrasse 6**
**A-4020 Linz (AT)**
Erfinder: **KLINAR, Gottfried**
**Alpenstrasse 33**
**A-8707 Leoben (AT)**
Erfinder: **HANKE, Reinhart**
**Annaberggasse 2**
**A-8700 Leoben (AT)**

(74) Vertreter: **Haffner, Thomas M., Dr. et al**
**Patentanwaltskanzlei Dipl.-Ing. Adolf**
**Kretschmer Dr. Thomas M. Haffner**
**Schottengasse 3a**
**A-1014 Wien (AT)**

EP 0 216 872 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung bezieht sich auf eine Einrichtung zum kontinuierlichen Einbringen oder Ausbringen bzw. Adsorption und Desorption von Gasen in oder aus Flüssigkeiten, insbesondere von Substraten in biologischer Umsetzung, mit einem in die Flüssigkeit eintauchenden Rotor, sowie eine Verwendung einer derartigen Einrichtung für aerobe und anaerobe Umsetzungen von Substraten.

Unter Einbringen bzw. Ausbringen von Gasen in Flüssigkeiten soll im folgenden auch jede kontinuierliche Absorption bzw. Desorption mit oder ohne chemische Umsetzung verstanden werden.

Für die Belüftung bzw. Begasung von Flüssigkeiten ist es bereits bekannt, eine Reihe von Düsen unterhalb der Flüssigkeitsoberfläche eintauchen zu lassen. Es ist weiters bereits bekannt, für die bessere Durchmischung rotierende Flügelräder vorzusehen.

Im Zusammenhang mit der biologischen Umsetzung von Substraten ist es beispielsweise aus der DE-A-26 25 230 für die Behandlung von Abwässern bekanntgeworden, Mikroorganismen auf rotierenden Scheiben anzuordnen, welche zyklisch in das umzusetzende Substrat eintauchen und aus diesem Substrat wieder herausgehoben werden. Mit derartigen Einrichtungen wurde bereits der Vorteil erzielt, daß die Mikroorganismen kurzfristig sich unter vom Substrat verschiedenen Bedingungen wieder regenerieren konnten. Auf ähnliche Weise konnte bei Verwendung von Hefe zur Gärung ein Regenerieren der Hefe an sauerstoffhaltigen Gasen zwischengeschaltet werden, bevor die Hefe neuerlich in das zu vergärende Substrat eintauchte.

Aus der US-A-4 211 647 ist weiters ein anaerobes Verfahren zur Behandlung von Abwässern bekanntgeworden, wobei in einem quadratischen Querschnitt aufweisenden Gehäuse auf eine drehbare Welle normal stehende Scheiben in die zu behandelnde Flüssigkeit eintauchen, wobei an den rotierenden Scheiben wiederum Mikroorganismen anhaften. Die Flüssigkeit wird dabei durch die in einem Gehäuse angeordnete Einrichtung hindurchgepumpt. Aus der FR-A-1 320 157 ist in diesem Zusammenhang ein rotierendes Filter bekanntgeworden, an dessen bürstenförmiger Außenfläche Mikroorganismen anhaften. Aus der FR-A-2 264 584 ist eine Einrichtung zum Belüften von Flüssigkeiten bekanntgeworden, bei welcher an einer drehbaren Welle Scheiben schwenkbar angeordnet sind und die Welle in ein zu behandelnde Flüssigkeit enthaltendes Becken teilweise eingetaucht wird. Nachteilig bei diesen bekannten Ausbildungen ist die Tatsache, daß neben der Antriebsenergie für die Drehbewegung der Scheiben oder Filter jeweils eigene Vorrichtungen zur Umwälzung bzw. Aufrechterhaltung einer Strömung der zu behandelnden Flüssigkeit vorgesehen sein müssen und/oder für eine Bewegung einer Vorrichtung zur Belüftung bei Anordnung in einem Becken zum Überstreichen der gesamten Oberfläche Sorge getragen werden muß.

Die Erfindung zielt nun darauf ab, eine Einrichtung der eingangs genannten Art zu schaffen, mit welcher mit geringstem Energieaufwand ein wirksamer Stoffaustausch sichergestellt werden kann und andererseits eine Substratbewegung sichergestellt werden kann, welche die jeweilige Biozönose ohne nennenswerte Diffusionswege unmittelbar mit den umzusetzenden Substraten und Nährsubstraten in Verbindung setzen läßt. Zur Lösung dieser Aufgabe besteht die Erfindung im wesentlichen aus einer Einrichtung zum Einbringen oder Ausbringen von Gasen in oder aus Flüssigkeiten, insbesondere von Substraten in biologischer Umsetzung, mit einem in die Flüssigkeit eintauchenden Rotor, bei welcher der Rotor um eine zur Oberfläche der Flüssigkeit im wesentlichen parallele Achse in einem geschlossenen rohrförmigen Behälter rotierbar gelagert ist, der Rotor in Achsrichtung nebeneinander quer zur Achse orientierte, mit dem Rotor drehfest verbundene Scheiben aufweist, die mit Borsten ausgestattet oder aus porösem, insbesondere geschäumtem Material, Drahtgittern, Maschendrahtgewebe oder Streckmetall ausgebildet sind, wobei in Achsrichtung nebeneinander liegende Scheiben in wenigstens einem die Achse enthaltenden Schnitt abwechselnd einen Winkel von größer und kleiner als 90° einschließen, der Umfang der Scheiben in einem, einen Drosselquerschnitt definierenden Abstand vom Innenmantel des Rohres liegt und an das Rohr wenigstens ein Flüssigkeitszulauf, ein Flüssigkeitsablauf sowie wenigstens eine Gaszutrittsöffnung und eine Gasabzugsöffnung angeschlossen sind. Dadurch, daß der Rotor in einem geschlossenen rohrförmigen Behälter rotierbar gelagert ist, läßt sich eine gerichtete Strömung durch das Rohr hindurch aufrechterhalten und es läßt sich eine kontinuierliche Umsetzung mit jeweils frischem Substrat bzw. frischer Biomasse erzielen. Dadurch, daß der Rotor in Achsrichtung nebeneinander quer zur Achse orientierte, mit dem Rotor drehfest verbundene Scheiben aufweist, die mit Borsten ausgestattet oder aus porösem, insbesondere geschäumtem Material, Drahtgittern, Maschendrahtgewebe oder Streckmaterial ausgebildet sind, läßt sich an der Oberfläche dieser Scheiben eine entsprechende Biomasse adsorbieren. Bei einer derartigen Ausbildung der Oberfläche der Scheiben werden bei jeder Umdrehung der Scheiben nennenswerte Substratmengen in Achsrichtung durch die Scheiben hindurchgepreßt und in die Gegenrichtung wiederum zurückgespült, wodurch eine regelmäßige Spülung und Reinigung der Biozönose erfolgt. Gleichzeitig wird immer neues Substrat an die Mikroorganismen herangeführt, wodurch die Umsetzung bedeutend beschleunigt werden kann. Weiters ist vor allen Dingen die Tatsache, daß die Scheiben in Achsrichtung nebeneinanderliegend in wenigstens einem die Achse enthaltenden Schnitt abwechselnd einen Winkel von größer und kleiner als 90° einschließen und der Umfang der Scheiben in einem, einen Drosselquerschnitt definierenden Abstand vom Innenmantel des Rohres liegt, dafür verantwortlich, daß eine pulsierende Bewegung innerhalb des Rohres erzielt wird, bei welcher der Flüssigkeitsspiegel in benachbarten Abschnitten angehoben und abgesenkt werden kann oder aber im Falle von entsprechend angeordneten Überströmöffnungen in den Scheiben eine gerichtete Strömung aufrechterhalten werden kann. Die Maßnahme, die Scheiben quer und unter einem Winkel abweichend von 90° mit der Rotationsachse an einer Rotationsachse rotierbar zu lagern, führt hiebei in besonders wirksamer Weise zu einer charakteristischen Substratbewegung, die durch auf- und abwärtsgerichtete Strömungen sowie Querströmungen innerhalb des Rohres gekennzeichnet ist. Eben diese Strömungen bewirken ein Ansaugen von oberhalb des Flüssigkeitsspiegels angebotenem Gas in die Flüssigkeit und es wird auf besonders schonende Weise und mit geringstem Energieaufwand eine Begasung der Flüssigkeit, insbesondere eine Belüftung, erzielt. Der geschlossene rohrförmige, in Kammern unterteilte Behälter erlaubt in einfacher Weise den gasdichten Abschluß der Einrichtung, so daß verschiedenste Gasatmosphären oberhalb des Flüssigkeitsspiegels eingebracht werden können und durch die Flüssigkeit auf Grund ihrer Bewegung, insbesondere ihrer Oberflächenbewegung, angesaugt werden können. An das Rohr ist hiebei wenigstens ein Flüssigkeitszulauf, ein Flüssigkeitsablauf sowie wenigstens eine Gaszutrittsöffnung und eine Gasabzugsöffnung angeschlossen. Die auf Grund ihrer Orientierung zur Achse eine Trommelbewegung ausübenden Scheiben üben ein gewisses Ausmaß an Schereffekt auf die Flüssigkeit aus, welches gleichfalls unter Ausschluß nennenswerter Diffusionswege eine rasche Umsetzung mit einer an den Scheiben anhaftenden Biozönose sicherstellt.

Gleichzeitig erlaubt die erfindungsgemäße Ausbildung das abwechselnde Regenerieren von biologischem Material durch Austauchen aus der Flüssigkeit, wobei durch Wahl der entsprechenden Gasatmosphäre eine oxidative Regenerierung, beispielsweise im Falle von Hefen, oder eine Regenerierung unter einer jeweils angepaßten Gasatmosphäre erfolgen kann.

In benachbarten Bereichen des Behälters läßt sich ohne weiteres alternativ eine aerobe und eine anaerobe Umsetzung durchführen, wofür es beispielsweise genügt, daß der rohrförmige Behälter in Achsrichtung durch im wesentlichen normal zur Achse orientierte Trennwände in Kammern unterteilt ist, welche Drosselquerschnitte für den Materialtransport zwischen benachbarten Kammern aufweisen. Diese Drosselquerschnitte für den Materialtransport zwischen benachbarten Kammern liegen unterhalb der Oberfläche der Flüssigkeit und es wird dadurch ein gasdichter Abschluß erzielt. Es kann somit in benachbarten Abschnitten eine unterschiedliche Gasatmosphäre eingesetzt werden. Der Transport des flüssigen Mediums kann durch externe Pumpen bewerkstelligt werden. Die Scheiben können im wesentlichen runden oder elliptischen Umriß aufweisen. Vorzugsweise ist die Füllstandshöhe in den Kammern zweckmäßig auf 20 - 60 % des lichten Durchmessers des rohrförmigen Behälters begrenzt.

Die Gaszuführ- und Gasaustrittsöffnungen können in einfacher Weise oberhalb der maximalen Füllstandshöhe nahe den beiden Stirnseiten des rohrförmigen Behälters angeschlossen sein. Je nach Strömungsrichtung der Flüssigkeit kann im Gegenstrom im Gleichstrom oder im Kreuzstrom mit der Flüssigkeit eine Begasung durchgeführt werden. Im Falle der Anordnung von Trennwänden kann die Ausbildung so getroffen sein, daß die Trennwände der einzelnen Kammern ortsfest angeordnet sind und Überströmöffnungen unterhalb der Füllstandshöhe, vorzugsweise im Bereich nahe dem Innenmantel des Behälters, angeordnet sind, und daß an jede Kammer gesonderte, quer zur Rotationsachse angeordnete Gaseintrags- und Gasaustragsöffnungen angeschlossen sind.

Die Scheiben können mit Mikroorganismen oder Biomasse beschichtet sein, um eine biologische Umsetzung durchzuführen. Biologische Umsetzungen können im Rahmen der Abwasserreinigung in Kläranlagen als anaerobe und aerobe Umsetzungen durchgeführt werden. Im Falle der Faulung von Klärschlamm wird zumeist ein anaerober Prozeß durchgeführt, bei welchem Methan, $CO_2$, Klärgas od. dgl. entsteht und aus den jeweiligen Kammern eines derartigen Behälters abgezogen werden kann. Eine Reihe von anaerob umsetzenden Mikroorganismen kann seine Aktivität erheblich erhöhen, wenn zwischenzeitlich eine aerobe Phase eingeschaltet wird, welche durch Austauchen der Bakterien in eine entsprechende Gasatmosphäre oder aber durch Lufteintrag in die Flüssigkeit zumindest in einzelnen Verfahrensstufen bewirkt werden kann. Bei der Essiggärung läuft in der Regel ein aerober Vorgang ab, welcher den Eintrag von Luft erfordert. Bei der kontinuierlichen Alkoholgärung unter Einsatz von mit Zucker angereicherten Hefen verläuft die eigentliche Umsetzung anaerob. Die Effizienz der Hefe kann jedoch wesentlich gesteigert werden, wenn diese zyklisch einer Belüftung unterworfen wird.

Mit Vorteil kann der Behälter mit Heiz- oder Kühleinrichtungen ausgestattet sein.

In besonders vorteilhafter Weise wird die erfindungsgemäße Einrichtung zum Belüften von Flüssigkeiten, insbesondere für aerobe Umsetzungen, verwendet. Die Einrichtung kann jedoch ebenso zum kontinuierlichen Gären von Alkohol aus vergärbarem Substrat oder generell für anaerobe Umsetzungen wie Faulung von Klärschlamm od. dgl. eingesetzt werden.

Weiters kann die erfindungsgemäße Einrichtung zum Ausgasen bei anaeroben Umsetzungen, insbesondere zur Abtrennung von $CO_2$, oder zum schonenden Mischen von Substrat und Biomasse verwendet werden.

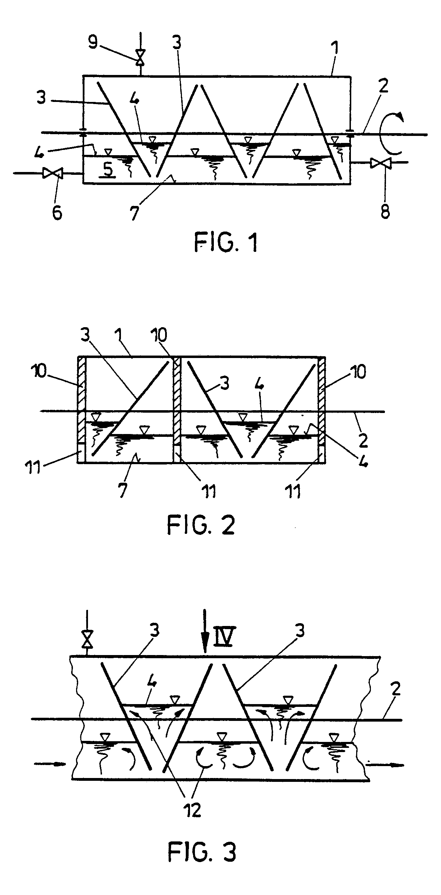

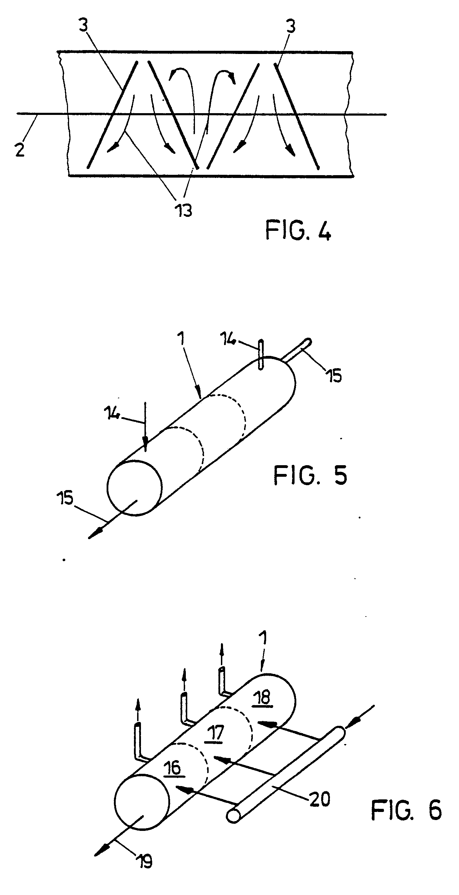

Die Erfindung wird nachfolgend an Hand von in der Zeichnung dargestellten Ausführungsbeispielen näher erläutert. In dieser zeigen: Fig. 1 eine

schematische Seitenansicht einer erfindungsgemäßen Einrichtung, Fig. 2 eine Teilansicht analog der Fig. 1 mit zusätzlichen Trennwänden, Fig. 3 und 4 die Strömungsverhältnisse in der flüssigen Phase in der Seitenansicht bzw. der Draufsicht entsprechend dem Pfeil IV der Fig. 3, Fig. 5 eine perspektivische Darstellung einer Mehrkammer-ausbildung mit Gleich- oder Gegenstrombelüf-tung, und Fig. 6 eine Ausbildung mit Trennwänden und einer Querstrombegasung bzw. -belüftung.

In Fig. 1 ist ein rohrförmiger Behälter 1 dargestellt, in welchem eine Welle 2 drehbar gelagert ist. Die Welle 2 trägt eine Mehrzahl von Scheiben 3, welche in der in Fig. 1 dargestellten Seitenansicht bzw. dem in Fig. 1 dargestellten Axialschnitt abwechselnd einen Winkel von größer und kleiner als 90° mit der Welle 2 einschließen. Die entsprechenden Winkel sind hiebei abwechselnd entgegengesetzt gleich. Die Scheiben 3 tauchen in den Flüssigkeitsspiegel 4 einer Flüssigkeit 5 ein, wobei der Zulauf von Flüssigkeit über ein Absperrventil 6 regelbar ist. Die Innenwand des rohrförmigen Behälters ist mit 7 bezeichnet und der Außenumfang der Scheiben 3, welche bei einem Rohr mit kreisrundem Durchmesser im wesentlichen runde oder elliptische Umrißform aufweisen, liegt in geringem Abstand vom Innenmantel 7 des rohrförmigen Behälters 1. Bei jeder Umdrehung der Scheiben 3, gemeinsam mit der Welle 2, erfolgt eine abwechselnde Anhebung und Absenkung des Flüssigkeitsspiegels 4, sofern der Drosselquerschnitt der zwischen dem Umfang der Scheiben 3 und der Innenwand 7 des Behälters verbleibt, hinreichend klein ist. Der Abfluß der Flüssigkeit wird über ein Absperrventil 8 geregelt. Die Gasatmosphäre kann je nach den Erfordernissen über ein Ventil 9 zugeführt oder abgeführt werden. Da der Behälter 1 druckfest ausgebildet ist, läßt sich die Anordnung auch unter unteratmosphärischem Druck betreiben.

Bei der Darstellung nach Fig. 2 sind zusätzlich Trennwände 10 zwischen benachbarten Kammern vorgesehen, welche Überströmöffnungen 11 unterhalb des Flüssigkeitsspiegels 4 aufweisen.

In Fig. 3 und 4 sind die Strömungsverhältnisse in der flüssigen Phase näher erläutert. Die Fig. 3 entspricht hiebei der Ansicht nach Fig. 1 und die Anhebung bzw. Absenkung des Flüssigkeitsspiegels in benachbarten Kammern führt zu einer entsprechenden zur Oberfläche und von der Oberfläche weg gerichteten Strömung, wie sie durch die Pfeile 12 näher veranschaulicht ist. In Fig. 4 ist die entsprechende Draufsicht auf die Anordnung nach Fig. 2 dargestellt und durch die Pfeile 13 sind die sich ergebenden Querströmungen bei jeder Umdrehung der Scheiben 3 symbolisiert.

Die Scheiben 3 sind für die biologische Umsetzung mit Mikroorganismen beladen und weisen zu diesem Zweck eine rauhe Oberfläche auf. Bevorzugt sind Scheiben aus Drahtgittern oder ·Streckmetall, welche einen Drosselwiderstand in Achsrichtung darstellen. Bei derartigen Ausbildungen ist die Anhebung bzw. Absenkung des Flüssigkeitsspiegels in benachbarten Kammern selbstverständlich nicht ausgeprägt. Auf Grund der in Fig. 3 und 4 veranschaulichten Strömungsverhältnisse erfolgt aber eine in Achsrichtung die Scheiben 3 durchsetzende Strömungsbewegung, wodurch ein intensiver Kontakt des Substrates mit den Mikroorganismen erfolgt.

In Fig. 5 ist ein Mehrkammerreaktor perspektivisch dargestellt, wobei die Gaseintrags- bzw. -austragsöffnungen mit 14 bezeichnet sind. Die Flüssigkeit wird über Leitungen 15 zu- und abgeführt. Je nach der Zuführungsrichtung des Gases kann die Gasführung im Gegenstrom im Gleichstrom oder im Gleichstrom zur Strömungsrichtung der Flüssigkeit erhalten werden.

Bei der Ausbildung nach Fig. 6 wird ein Querstrom gewählt, wobei zwischen den einzelnen Abschnitten 16, 17 und 18 des rohrförmigen Behälters 1 entsprechende Trennwände 10, wie in Fig. 2 dargestellt, vorgesehen sind. Diese Trennwände 10 weisen Überströmöffnungen 11 unterhalb des Flüssigkeitsspiegels auf und die Gasströmung erfolgt für jede der einzelnen Kammern 16, 17 und 18 jeweils quer zur Strömungsrichtung der Flüssigkeit, welche durch den Pfeil 19 symbol-siert ist. Den einzelnen Abschnitten können unterschiedliche oder gleiche Gase zugeführt werden. Bei der Darstellung nach Fig. 6 wird das Gas über eine Sammelleitung 20 und damit jedem Abschnitt das gleiche Gas zugeführt.

**Patentansprüche**

1. Einrichtung zum Einbringen oder Ausbringen von Gasen in oder aus Flüssigkeiten, insbesondere von Substraten in biologischer Umsetzung, mit einem in die Flüssigkeit eintauchenden Rotor, bei welcher der Rotor um eine zur Oberfläche der Flüssigkeit (4) im wesentlichen parallele Achse in einem geschlossenen rohrförmigen im wesentlichen horizontalen Behälter (1) rotierbar gelagert ist, der Rotor in Achsrichtung nebeneinander quer zur Achse orientierte, mit dem Rotor drehfest verbundene Scheiben (3) aufweist, die mit Borsten ausgestattet oder aus porösem, insbesondere geschäumtem Material, Drahtgittern, Maschendrahtgewebe oder Streckmetall ausgebildet sind, wobei in Achsrichtung nebeneinander liegende Scheiben (3) in wenigstens einem die Achse enthaltenden Schnitt abwechselnd einen Winkel von größer und kleiner als 90° einschließen, der Umfang der Scheiben (3) in einem, einen Drosselquerschnitt definierenden Abstand vom Innenmantel (7) des Rohres (1) liegt und an das Rohr wenigstens ein Flüssigkeitszulauf, ein Flüs-sigkeitsablauf sowie wenigstens eine Gaszutritts-öffnung und eine Gasabzugsöffnung ange-schlossen sind.

2. Einrichtung nach Anspruch 1, dadurch gekennzeichnet, daß die Scheiben (3) elliptischen Umriß aufweisen.

3. Einrichtung nach Anspruch 1 oder 2, dadurch gekennzeichnet, daß der rohrförmige Behälter (1) in Achsrichtung durch, vorzugsweise im wesentlichen normal zur Achse orientierte, Trennwände (10) in Kammern (16, 17, 18) unterteilt ist, welche Drosselquerschnitte (11) für den Materialtransport zwischen benachbarten Kammern (16, 17, 18) aufweisen.

4. Einrichtung nach Anspruch 1 bis 3, dadurch gekennzeichnet, daß die Füllstandshöhe auf 20 - 60 % des lichten Durchmessers des rohrförmigen Behälters (1) begrenzt ist.

5. Einrichtung nach einem der Ansprüche 1 bis 4, dadurch gekennzeichnet, daß die Gaszuführ- und Gasaustrittsöffnungen oberhalb der maximalen Füllstandshöhe nahe den beiden Stirnseiten des rohrförmigen Behälters (1) angeschlossen sind.

6. Einrichtung nach einem der Ansprüche 1 bis 5, dadurch gekennzeichnet, daß die Scheiben (3) mit Mikroorganismen oder Biomasse beschichtet sind.

7. Einrichtung nach einem der Ansprüche 1 bis 6, dadurch gekennzeichnet, daß der rohrförmige Behälter (1) mit Heiz- oder Kühleinrichtungen ausgestattet ist.

8. Verwendung einer Einrichtung nach einem der Ansprüche 1 bis 7 zum Belüften von Flüssigkeiten für aerobe Umsetzungen.

9. Verwendung einer Einrichtung nach einem der Ansprüche 1 bis 7 zur Herstellung von Alkohol aus vergärbarem Substrat durch Gärung.

## Revendications

1. Agencement d'introduction de gaz dans des liquides ou de séparation de gaz et de liquides, notamment de substrats en cours de transformation biologique, avec un rotor plongeant dans le liquide, dans lequel, le rotor est monté à rotation autour d'un axe sensiblement parallèle à la surface (4) du bain de liquide, à l'intérieur d'un réservoir tubulaire fermé (1) sensiblement horizontal, et le rotor comporte des disques (3) orientés transversalement à l'axe et agencés côte à côte selon la direction axiale en étant liés en rotation au rotor, ces disques étant équipés de poils ou étant réalisés en matériaux poreux, notamment sous forme de mousse, d'armatures métalliques, de treillis de fils métalliques, ou de métal déployé, les disques (3) voisins en direction axiale, formant avec l'axe, dans au moins un plan de coupe renfermant l'axe, un angle alternativement supérieur et inférieur à 90°, la périphérie des disques (3) étant située à une distance de l'enveloppe interne (7) du tube (1) de manière à définir une section d'étranglement, au moins une alimentation et un écoulement d'évacuation de liquide, ainsi qu'au moins une bouche d'arrivée de gaz et une bouche d'extraction de gaz étant raccordés sur le tube.

2. Agencement selon la revendication 1, caractérisé en ce que les disques (3) présentent un contour elliptique.

3. Agencement selon la revendication 1 ou 2, caractérisé en ce que le réservoir tubulaire (1) est subdivisé, selon la direction axiale, en chambres (16, 17, 18) par des cloisons de séparation (10) qui de préférence sont sensiblement perpendiculaires à l'axe, et qui présentent des passages formant sections d'étranglement (11) pour permettre de véhiculer le matériau entre les chambres voisines (16, 17, 18).

4. Agencement selon les revendications 1 à 3, caractérisé en ce que le niveau de remplissage est limité à 20 à 60% du diamètre de passage du réservoir tubulaire (1).

5. Agencement selon l'une des revendications 1 à 4, caractérisé en ce que les bouches d'entrée et de sortie de gaz sont raccordées au-dessus du niveau de remplissage maximal, à proximité des deux extrémités frontales du réservoir tubulaire (1).

6. Agencement selon l'une des revendications 1 à 5, caractérisé en ce que les disques (3) sont revêtus de micro-organismes ou de biomasse.

7. Agencement selon l'une des revendications 1 à 6, caractérisé en ce que le réservoir tubulaire (1) est équipé de dispositifs de chauffage ou de réfrigération.

8. Application d'un agencement selon l'une des revendications 1 à 7, à l'aérage de liquides en vue de transformations aérobies.

9. Application d'un agencement selon l'une des revendications 1 à 7, à la fabrication d'alcool par fermentation, à partir de substrats fermentescibles.

## Claims

1. Device for introducing gases, in particular substrates undergoing a biological conversion, into or extracting them from liquids, having a rotor dipping into the liquid, in which the rotor is mounted in a closed, tubular essentially horizontal container (1) rotatably about an axis essentially parallel to the surface of the liquid (4), the rotor has plates (3) which are oriented next to one another in the axial direction, transverse to the axis, are non-rotatably connected to the rotor, and are fitted with bristles or constructed from a porous, in particular foamed material, wire gratings, wire-netting cloth or expanded metal, plates (3) lying next to one another in the axial direction enclosing, in at least one cross-section containing the axis, alternately an angle of greater than and less than 90°, the circumference of the plates (3) lies at a distance from the inside casing (7) of the tube (1) that defines a restricted cross-section, and at least one liquid inlet, one liquid outlet, as well as at least one gas-supply opening and one gas-vent opening are connected to the tube.

2. Device according to Claim 1, characterized in that the plates (3) have an elliptical contour.

3. Device according to Claim 1 or 2, characterized in that the tubular container (1) is divided, in the axial direction, into chambers (16, 17, 18) by partition walls (10) preferably oriented essentially perpendicular to the axis and which have restricted cross-sections (11) for the transport of material between adjacent chambers (16, 17, 18).

4. Device according to Claims 1 to 3, characterized in that the fill level height is limited to 20-60% of the clear diameter of the tubular container (1).

5. Device according to one of Claims 1 to 4, characterized in that the gas-supply and gas-exit openings are connected above the maximum fill level height close to the two end sides of the tubular container (1).

6. Device according to one of Claims 1 to 5, characterized in that the plates (3) are coated with microorganisms or biomass.

7. Device according to one of Claims 1 to 6, characterized in that the tubular container (1) is fitted with heating or cooling devices.

8. Use of a device according to one of Claims 1 to 7, for aerating liquids for aerobic conversions.

9. Use of a device according to one of Claims 1 to 7, for producing alcohol from a fermentable substrate by fermentation.

FIG. 1

FIG. 2

FIG. 3

EP 0 216 872 B1

FIG. 4

FIG. 5

FIG. 6

2